# EUROPÄISCHE PATENTSCHRIFT

(11) **EP 0 455 034 B1**
(45) Veröffentlichungstag und Bekanntmachung des Hinweises auf die Patenterteilung: **04.01.1995**
(21) Anmeldenummer: 91105954.1
(22) Anmeldetag: 15.04.1991
(51) Int. Cl.: A61K 35/78

(54) **Knoblauchsaft zum Einnehmen**
Garlic juice to be taken
Jus d'ail à consommer

(30) Priorität: 27.04.1990 DE 4013499
(43) Veröffentlichungstag der Anmeldung: 06.11.1991
(73) Patentinhaber: Berchtold, Dieter, D-78224 Singen (DE)
(72) Erfinder: Berchtold, Dieter, D-78224 Singen (DE)
(74) Vertreter: Weiss, Peter, Dr. rer.nat.

(56) Entgegenhaltungen:
- WPIL/DERWENT, AN=84-084185, Derwent Publications Ltd, London, GB;& JP-A-54 098 310 (T. KOIKE)
- WPI/DERWENT, AN=76-19341X, Derwent Publications Ltd, London, GB;& JP-A-51 009 751 (K. OSAJIMA)

## Beschreibung

Die Erfindung betrifft einen Knoblauchsaft zum Einnehmen, insbesondere zur Stärkung des Herz- und Kreislaufsystemes sowie des Immunsystems.

Knoblauchpräparate wie auch Vitaminpräparate sind weit verbreitet und werden in den verschiedensten Darreichungsformen angeboten. Sie gibt es beispielsweise als Tabletten, als Kapseln, als Dragées, als Lutschbonbons, als Säfte oder als Sirup um nur einige zu nennen.

Alle diese Präparate dienen einer Stärkung und Aufrechterhaltung der körperlichen Leistungsfähigkeit bzw. der Gesundheit, indem vor allem das Herz-Kreislaufsystem einerseits sowie das Immunsystem andererseits gestützt wird. Die Stützung und Stärkung des Herz-Kreislaufsystemes erfolgt dabei vor allem durch die Wirkstoffe des Knoblauchs.

Leider haben diese Präparate trotz allem den Nachteil, daß ihnen, da sie lagernd gehalten werden müssen, in den meisten Fällen konservierende Stoffe beigemengt sind und daß sie, aufgrund ihrer eher komplizierten Herstellung relativ teuer sind. Des weiteren haben vor allem die Knoblauchpräparate den Nachteil, daß sie aufgrund einer schlechten Verträglichkeit wieder aufstoßen unter Begleitung des dem Knoblauch üblichen Beigeschmackes. Anderer Präparate sorgen für eine entsprechende Ausdünstung durch die Poren, was ebenfalls zu einer unerwünschten und unangenehmen Geruchsbildung führt.

Der Erfinder hat sich nun zur Aufgabe gestellt, ein Präparat zu schaffen, welches das Herz-Kreislauf-System besser stützt, eine Immunsystemstärkung bewirkt und welches nach Einnahme weder einen üblen Nachgeschmack noch Nachgeruch mit sich bringt.

Zur Lösung dieser Aufgabe führt, daß der Saft außer Knoblauch auch Honig und destilliertes Wasser enthält.

Bei einem solchen Knoblauch-Vitaminsaft fehlt jeglicher Konservierungsstoff, die Haltbarkeit - zwar begrenzt - erfolgt durch das vorhergehende Pasteurisieren bzw. Abkochen des Wassers und durch eine kühle Lagerung. Bevorzugt wird ein Zusatz von 5 - 20% Knoblauch und 10 - 20% Honig.

In einem bevorzugten Ausführungsbeispiel enthält der Saft noch zusätzlich 15 - 25% Zitrone sowie ggfs. 10 - 20% gerösteten Dinkel.

Der Knoblauch wie auch ggfs. die Zitronen werden geschält und in kleine Würfel geschnitten und anschließend unter Beigabe einer entsprechenden Menge von Honig in abgekochtem Wasser während 5 Tagen angestellt.

Die Zitrone, als Lieferant des wichtigen Vitamin C entzieht dem Knoblauch einerseits die Wirksubstanzen und führt andererseits zu einer annähernden Homogenisierung des gesamten Präparates. Die Zitrone sowie der Knoblauch sind nur noch als Satz in einer gelblich trüben Flüssigkeit sichtbar.

Die Beigabe von Honig verfeinert den Geschmack und bereichert den Saft zusätzlich durch seine Wirkstoffe, die ebenfalls zur Stärkung des Immunsystems beitragen. Ferner bindet Honig den Geschmack des Knoblauchs. Ein solches Präparat kann täglich in einer kleinen Menge, vorzugsweise zum Frühstück zur Prophylaxe bzw. Prävention von insbesondere Erkältungs- und Kreislaufkrankheiten eingenommen werden.

Neben einer guten Verträglichkeit und einem hohen Wirkungsgrad besteht der Haupterfindungsgedanke aber darin, daß dieser Saft in Verbindung mit dem Trinken von Kaffee absolut keinen Nachgeschmack bzw. Nachgeruch oder Ausdünstungen od. dgl. aufweist. Diese Eigenschaft des Präparates läßt seine ungenierte Einnahme auch dann zu, wenn im Anschluß daran Kontakt zu anderen Personen besteht, wo ein Nachgeschmack insbesondere ein Knoblauchgeruch sehr unangenehm oder störend sein könnte.

In einem weiteren Ausführungsbeispiel der Erfindung ist dem Knoblauch-Vitaminsaft gerösteter Dinkel beigestetzt. Der Dinkel hat bei dieser Ausführungsform die Eigenschaft, nach Einnahme im Magendarmtrakt zu quellen und so als Wassertransporteur zu dienen. Durch diese Eigenschaft wird eine Regulierung der Darmperistaltik herbeigeführt bzw. eine Normalisierung der Verdauungstätigkeit. Somit sind in diesem Ausführungsbeispiel die Wirkungskomponenten im Bezug auf Herz- und Kreislaufstärkung sowie auf Stärkung des Immunsystems auf die Regulierung der Verdauung erweitert.

Der eben genannte Knoblauch-Vitamin-Saft hat einen sehr starken Geruch. Dieser Geruch kann dadurch gemindert werden, daß dem Gemisch während des Anstellens Apfelstücke, insbesondere geschälte Apfelstücke, zugesetzt werden. Erstaunlicherweise hat sich herausgestellt, daß diese Apfelstücke den Geruch von Knoblauch weitgehend binden. Beim fertigen Trinksaft geschieht die Bindung des Geruchs durch den Zusatz von einigen Tropfen Apfelsaft.

Insgesamt wird mit dieser Erfindung ein Mittel hergestellt, das auf biologisch natürlichen und einwandfreien Produkten basiert, einen hohen Wirkungsgrad erzielt ohne die üblichen Nebeneffekte, wie Aufstoßen, Ausdünsten od. dgl., zu besitzen und gleichzeitig kostenkünstig in der Herstellung ist.

## Patentansprüche

1. Knoblauchsaft zum Einnehmen, insbesondere zur Stärkung des menschlichen Herz- und Kreislaufsystems sowie des Immunsystems,
dadurch gekennzeichnet,
daß der Saft außer dem Knoblauch auch Honig und destilliertes Wasser enthält.

2. Saft nach Anspruch 1, dadurch gekennzeichnet, daß dem destillierten Wasser etwa 5 - 20% Knoblauch und 10 - 20% Honig zugesetzt sind.

3. Saft nach Anspruch 1 oder 2, dadurch gekennzeichnet, daß der Saft zusätzlich 15 - 25% Zitrone enthält.

4. Saft nach einem der Ansprüche 1 bis 3, dadurch gekennzeichnet, daß der Saft einen Zusatz von etwa 10 - 20% gerösteten Dinkel enthält.

5. Verfahren zum Herstellen eines Saftes zum Einnehmen, insbesondere zur Stärkung des menschlichen Herz- und Kreislaufsystems sowie des Immunsystems, dadurch gekennzeichnet, daß Wasser abgekocht und zusammen mit geschältem sowie geschnittenem Knoblauch und Honig angestellt und der Saft nach ca. einer Woche abgefiltert wird.

6. Verfahren nach Anspruch 5, dadurch gekennzeichnet, daß das Gemisch aus Wasser, Knoblauch, Honig, geschälter Zitrone und ggfs. geröstetem Dinkel angestellt wird.

7. Verfahren nach Anspruch 5 oder 6, dadurch gekennzeichnet, daß dem Gemisch während des Anstellens Apfelstücke, insbesondere geschälte Apfelstücke, zugesetzt werden.

## Claims

1. Garlic juice to be taken, in particular for strengthening the human cardiac and circulatory system and the immune system, characterised in that the juice also contains honey and distilled water in addition to the garlic.

2. Juice according to Claim 1, characterised in that about 5 - 20% of garlic and 10 - 20% of honey are added to the distilled water.

3. Juice according to Claim 1 or 2, characterised in that the juice additionally contains 15 - 25% of lemon.

4. Juice according to one of Claims 1 to 3, characterized in that the juice has an addition of about 10 to 20% of roast spelt.

5. Process for producing a juice to be taken, in particular for strengthening the human cardiac and circulatory system as well as the immune system, characterised in that water is boiled and is fermented with peeled and chopped garlic and honey and the juice is filtered after about 1 week.

6. Process according to Claim 5, characterised in that the mixture of water, garlic, honey, peeled lemon and optionally roasted spelt is fermented.

7. Process according to Claim 5 or 6, characterised in that pieces of apple, in particular peeled pieces of apple, are added to the mixture during fermentation.

## Revendications

1. Jus d'ail à consommer, notamment pour renforcer le coeur et le système circulatoire humain ainsi que le système immunitaire, caractérisé en ce que le jus contient non seulement du jus d'ail mais également du miel et de l'eau distillée.

2. Jus selon la revendication 1, caractérisé en ce qu'à l'eau distillée on ajoute entre 5 et 20% d'ail et 10 à 20 % de miel.

3. Jus selon la revendication 1 ou 2, caractérisé en ce qu'il contient en outre entre 15 et 25 % de citron.

4. Jus selon l'une des revendications 1 à 3, caractérisé en ce qu'il reçoit un additif d'épautre torréfié représentant 15 à 25%.

5. Procédé pour fabriquer un jus à consommer, notamment pour renforcer le coeur et le système circulatoire humein ainsi que le système immunitaire, caractérisé en ce qu'on fait bouillir l'eau et on laisse reposer avec de l'ail pelé et coupé et du miel puis on filtre le jus après environ une semaine.

6. Procédé selon la revendication 5, caractérisé en ce qu'on laisse reposer un mélange d'eau, d'ail, de miel, de citron pelé et le cas échéant d'épautre torréfié.

7. Procédé selon la revendication 5 ou 6, caractérisé en ce que l'on ajoute au mélange, pendant qu'il repose, des morceaux de pomme, notamment des morceaux de pommes pelés.
